(19) Europäisches Patentamt
European Patent Office
Office européen des brevets

(11) Publication number: **0 268 773 B1**


(12) # EUROPEAN PATENT SPECIFICATION


(45) Date of publication of patent specification: **15.04.92**

(51) Int. Cl.[5]: **G01N 33/569**, //G01N33/546, G01N33/549

(21) Application number: **87113783.2**

(22) Date of filing: **21.09.87**


(54) **Novel method for determination of antistreptolysin O and a kit used therefor.**


(30) Priority: **24.09.86 JP 225118/86**

(43) Date of publication of application:
**01.06.88 Bulletin 88/22**

(45) Publication of the grant of the patent:
**15.04.92 Bulletin 92/16**

(84) Designated Contracting States:
**AT BE CH DE ES FR GB GR IT LI LU NL SE**



(56) References cited:
**EP-A- 0 043 608**
**EP-A- 0 089 598**
**EP-A- 0 108 612**
**DE-B- 2 657 926**
**GB-A- 2 069 133**

**CHEMICAL ABSTRACTS, vol. 90, no. 2, February 1979, Columbus, OH (US); D.PRIGENT et al., p. 87, no. 67121s**

**CHEMICAL ABSTRACTS, vol. 83, no. 21, 24 November 1975, Columbus, OH (US); J.L.DUNCAN et al., p. 131, no. 173595q**



(73) Proprietor: **WAKO PURE CHEMICAL INDUSTRIES, LTD.**
**10, Doshomachi-3-chome**
**Higashi-ku Osaka(JP)**

(72) Inventor: **Kida, Masaaki**
**2-21, Nishinoshocho**
**Suita-shi(JP)**
Inventor: **Kitabata, Isako**
**Heiwaso 50 10-1, Minamitsukaguchicho-1-chome**
**Amagasaki-shi(JP)**
Inventor: **Kubotsu, Kazuhisa**
**Yamamotoryo 35 17-35, Daidominami-1-chome**
**Higashiyodogawa-ku Osaka(JP)**
Inventor: **Sakata, Yoshitsugu**
**36-7, Hiyoshidai-3-chome**
**Otsu-shi(JP)**

(74) Representative: **Baillie, Iain Cameron et al**
**c/o Ladas & Parry, Altheimer Eck 2**
**W-8000 München 2(DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUD OF THE INVENTION

1. FIELD OF THE INVENTION

This invention relates to a novel method for determination of antistreptolysin O (hereinafter abbreviated as ASO) value using liposomes, and a kit used therefor.

More particularly, this invention relates to a novel method for determining ASO value in a sample using liposomes encapsulating a labeling substance, and streptolysin O (hereinafter abbreviated as SLO) as antigen; and a kit used therefor. According to the novel determination method and kit of this invention, ASO value in a sample can be determined easily, rapidly and accurately and the determination can be automated.

2. RELATED ART STATEMENT

Determination of ASO value in serum is a widely used procedure for the diagnosis of a previous streptococcal infection. In detail, SLO is a hemolytic toxin produced by group A streptococci and has antigenicity. Therefore, when group A-streptococcal infections occur, antibody against SLO (that is ASO) value in serum is increased, and hence previous group A-streptococcal infections can be confirmed by detecting this increase.

Conventional methods for determining ASO value include the Rantz-Randall method and the micro-titer method which use human (O group), rabbit or sheep erythrocytes. In both of these methods, erythrocytes and SLO as antigen are used. ASO value is determined by measuring the amount of erythrocytes lysed, by utilizing a so-called neutralization reaction in which SLO reacts with ASO, i.e., an antibody in a sample serum, resulting in inactivation of the toxic activities such as hemolytic activity and cytolytic activity of SLO. However, in these methods, fresh erythrocytes of an animal or a human being which are very unstable are unavoidably needed, and therefore measured values tend to scatter and a complicated procedure is required. Furthermore, the reaction time is relatively long. Accordingly, these methods are very difficult to be automated.

On the other hand a determination method by immuno-nephelometry utilizing a kind of precipitation reaction has also been reported (Japanese Patent Application Kokai (Laid-Open) No. 25062/86). However, the degree of turbidity produced by the antigen-antibody reaction between SLO and ASO is much lower than that in other antigen-antibody reactions. Therefore, additives such as so-called agglutination promoters, etc. should be added. But since such additives precipitate also other proteins non-specifically, a determination system becomes complicated, and hence automation of said determination method involves many problems and no sufficiently accurate determination of ASO value can be achieved.

GB-A-2 069 133 discloses the use of immunoreactive liposomes in an immunoassay method but differs from the present invention in that it has not been described for the detection of ASO; the presence of complement factors is essential; and antigens or antibodies are always immobilised on the liposomes.

SUMMARY OF THE INVENTION

Accordingly, an object of this invention is to provide a novel determination method which uses neither unstable substances such as erythrocytes nor additives such as agglutination promoters, and permits simple, rapid and accurate determination of ASO value in a sample.

Another object of this invention is to provide a novel method for determining ASO value which can be automated.

Further another object of this invention is to provide a kit used for the novel determination method described abode.

Other and further objects and advantages of this invention will be apparent from the following description.

The objects and advantages of this invention described above can be achieved by the following novel determination method and kit.

This invention is a method for determining ASO value which comprises adding (a) liposomes encapsulating a labeling substance and (b) SLO as both antigen and lytic agent to a sample, measuring the amount of the labeling substance released by lysis of the liposome membrane by SLO, and thereby determining ASO value in the sample; and a kit for determining ASO value which comprises a suspension of liposomes encapsulating a labeling substance, and a solution of SLO.

The determination method of this invention utilizes the ability of SLO to lyse liposomes by its hemolytic activity and cytolytic activity. More particularly, the determination method of this invention comprises adding (a) liposomes encapsulating a labeling substance and (b) SLO to a serum sample; measuring the amount of the labeling substance released by lysis of the liposome membrane by SLO remaining after the antigen-antibody reaction between ASO in the sample and SLO added; and determining the ASO value in the sample from a standard calibration curve previously prepared by conducting determinations for standard samples in the same manner as described above.

The determination method of this invention is a simple, rapid and exact method for determining ASO value which can be automated.

BRIEF DESCRIPTION OF THE DRAWINGS

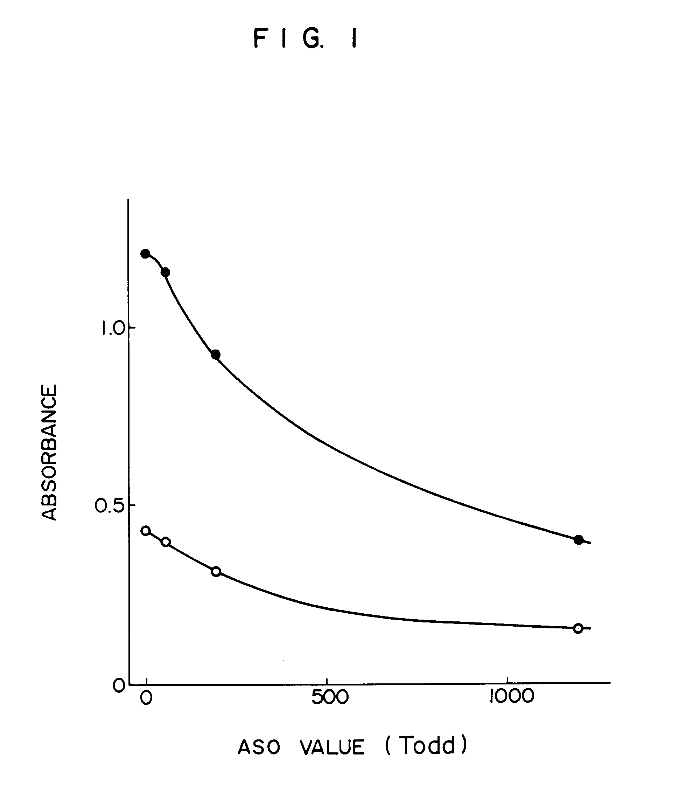

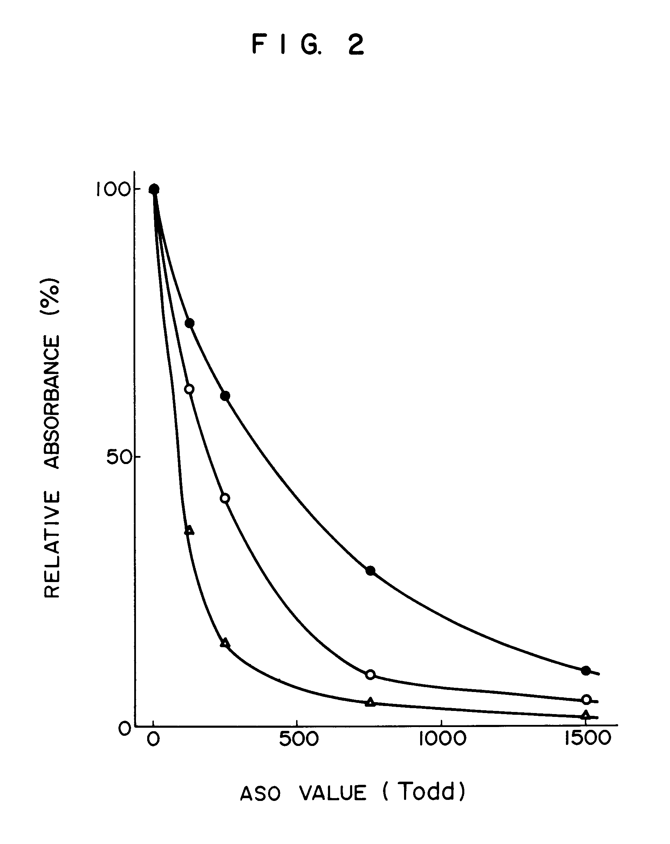

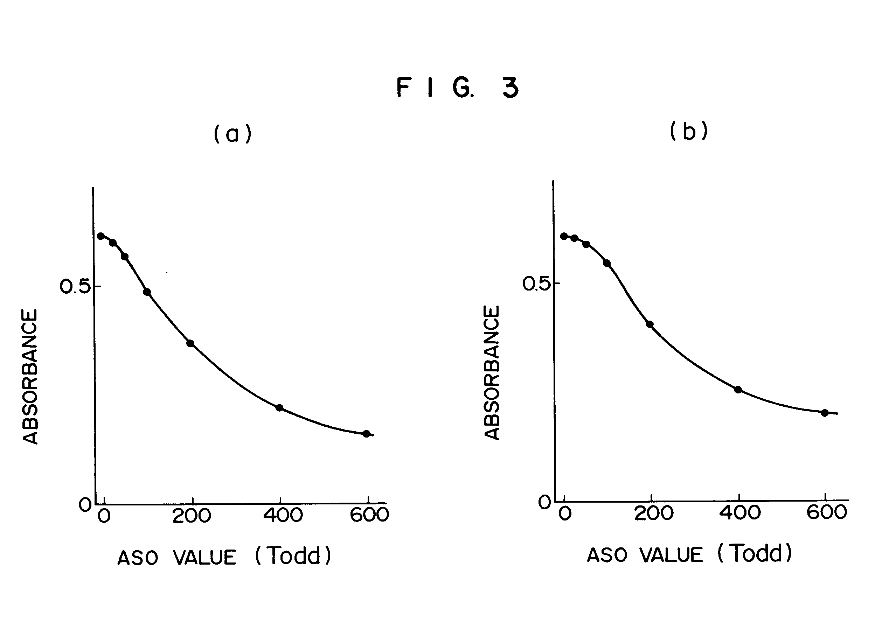

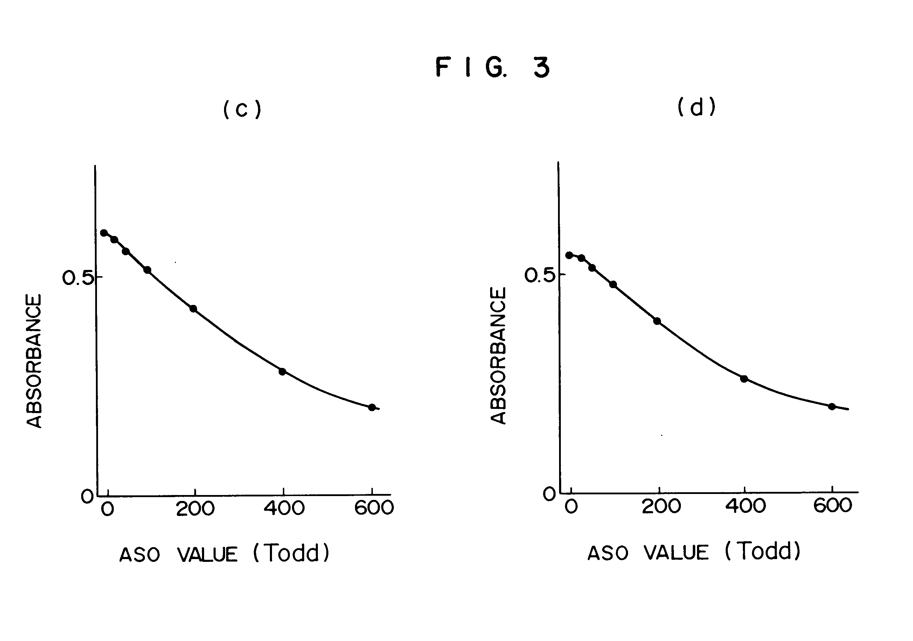

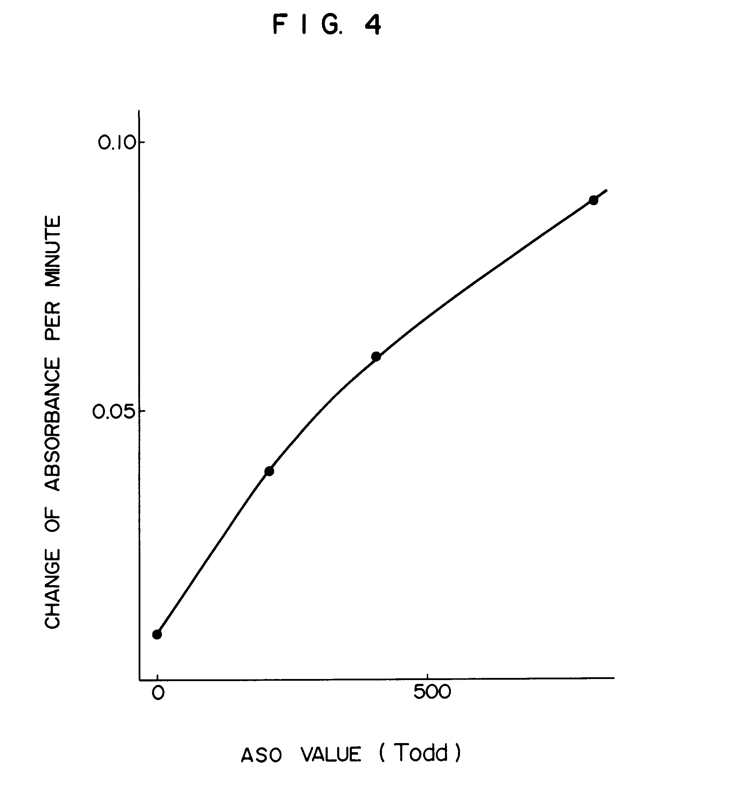

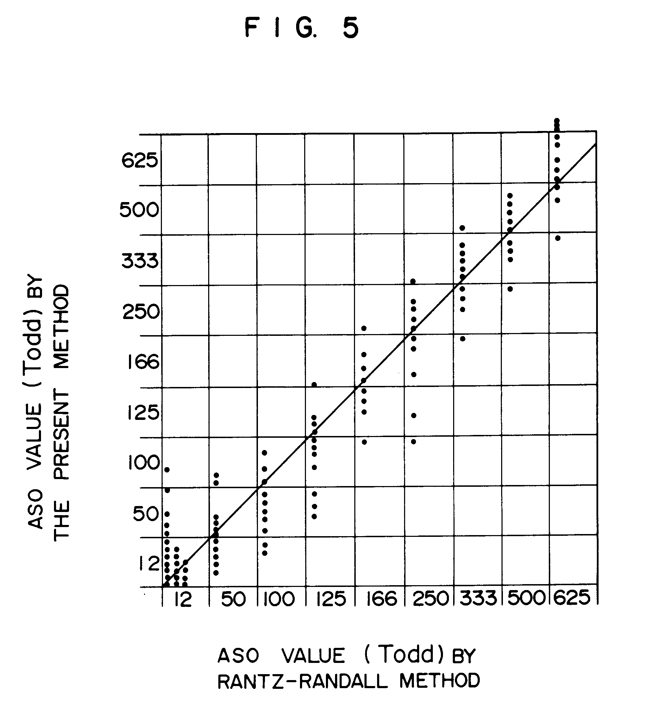

Fig. 1 shows the calibration curves obtained in Examples 1 and 2, the axis of abscissa refers to ASO value (Todd) and the axis of ordinate to absorbance at 410 nm. In Fig. 1 -○- shows the result obtained in Example 1 and -●- that obtained in Example 2. Fig. 2 shows the results obtained in Example 3, the axis of abscissa refers to the ASO value and the axis of ordinate to the relative absorbance determined by taking the absorbance (at 410 nm) at an ASO value of zero as 100 (i.e., an absorbance value determined by taking the absorbance measured for a sample containing no ASO) (%). In Fig. 2, -●- -○-, and -Δ- show the results obtained when the amount of SLO in a total reagent mixture (i.e., a mixture of a sample, a SLO solution and a liposome suspension) is 32 μg/ml, 16 μg/ml or 8 μg/ml, respectively. Fig. 3 (a) shows the result obtained in Example 4 and Fig. 3 (b), (c) and (d) those obtained in Example 5: (b), (c) and (d) shows the results obtained when the BSA content is 0.25%, 0.5% or 1.0%, respectively. In Fig. 3, the axis of abscissa refers to the ASO value (Todd) and the axis of ordinate to absorbance at 410 nm. Fig. 4 shows the calibration curve obtained in Example 6: the axis of abscissa refers to the ASO value (Todd) and the axis of ordinate to the change of absorbance per minute at 415 nm measured by using as a reference a reaction solution using physiological saline in place of a sample. Fig. 5 shows the correlation between the present method (the method of Example 6) and a conventional method (the Rantz-Randall method).

DETAILED DESCRIPTION OF THE INVENTION

As the liposome used in this invention, any one may be used, and there may be exemplified, for example, those made of mixtures of phospholipids or glycolipids and cholesterols or other lipids, and those made of mixtures of these materials and lipopolysaccharides (hereinafter abbreviated as LPS) or LPS-like compounds. As the phospholipids, there may be used any of conventional ones, for example, alkyl ester type phospholipids such as dipalmitoylphosphatidylcholine (DPPC), dimyristoylphosphatidylcholine (DMPC), distearoylphosphatidylcholine (DSPC), and the like; alkyl ether type phospholipids such as di-O-hexadecylphosphatidylcholine, di-O-octadecylphosphatidylcholine, didodecylphosphatidylcholine, and the like; and natural lecithins such as egg yolk lecithin, soybean lecithin, and the like. Among them, the alkyl ether type phospholipids are more preferred because in general they can form liposomes which are stable even at room temperature.

As LPS, there are usually used those derived from E. coli, those obtained by treatment by the Westphal method, etc.

As the LPS-like compounds, there may be exemplified compounds obtained by subjecting LPS to chemical modification, for example, compounds obtained by oxidation of the saccharide chain portion, compounds obtained by chemical treatment such as acetylation, succinylation, phthalylation or the like of the saccharide chain portion, and complexes of LPS and polymyxin having a high affinity for LPS or a carbocyanine type dye which is a basic photosensitive dye, etc.

As the glycolipids, conventional ones may be exemplified, and it is also possible to use conventional lipids other than those described above.

As the liposome usd in this invention, liposomes containing LPS or a LPS-like compound as a part of their constituents are preferred because they make it possible to increase the amount of a labeling substance to be encapsulated. As for such liposomes, the specification of European Patent Application No. 87107259,1 belonging to the state of the art under Art. 54(3) EPC only, and which the present inventors have previously filed is referred herein.

The labeling substance to be encapsulated in the liposomes includes, for example, enzymes such as alkaline phosphatase, β-galactosidase, glucose-6-phosphate dehydrogenase, etc.; coenzymes such as NAD, NADP, FAD, etc.; luminous compounds such as luminol, luciferin, etc.; fluorescent substances such as carboxyfluorescein, etc.; dyes such as 2,7-bis(2-arsonophenylazo)-1,8-dihydroxynaphtalene-3,6-disulfonic acid, etc.; sugars such as glucose, etc.; ionic compounds such as potassium dichromate, sodium dichromate, NaCl, etc.; and radical compounds such as nitroxide compounds, etc. But the labeling substance is not limited thereto, and any substance may be used so long as it can be encapsulated in the liposomes and can serve as a marker in determination of ASO value.

In this invention, the enzymes are particularly preferred as the labeling substance.

As a method for preparing the liposomes used in this invention, conventional preparation methods may be exemplified. That is to say, there may be exemplified all the well known methods for preparing liposomes, for example, the vortexing method [A.D. Bangham et al., J. Mol. Biol., 13, 238 (1965)], sonication methods [C. Huing et al., Biochemistry, 8, 344 (1969)], surfactant method [J.R. Slack et al., Biochem. Biophys. Acta., 323, 547 (1973)], reverse-phase evaporation method (REV method) [F. Szoka et al., Proc. Natl. Acad. Sci., U.S.A., 75, 4194 (1978)], ethanol infusion method, ether infusion method, pre-vesicle method [S.Batzri et al., Biochem. Biophs. Acta., 298, 1015 (1973)], French press extrusion method, $Ca^{2+}$ fusion method, annealing method, freeze-thaw-fusion method, freeze-drying methods, W/O/W emulsion method, methods such as the stable plurilamellar vesicle method (SPLV method) recently reported by S.M. Gruner et al. [Biochemistry, 24, 2833 (1985)], and methods for preparing liposomes called "giant liposomes" which have a large captured volume. The liposomes containing LPS or a LPS-like compound are prepared according to the process disclosed in the specification of European Patent Application No. 87107259,1, belonging to the state of the art under Art. 54(3) EPC only.

The labeling substance to be encapsulated in the liposomes is usually added at an optional step in the above-mentioned method for preparing liposomes.

The determination method of this invention comprises adding (a) a suspension of liposomes encapsulating the labeling substance and (b) a solution of SLO as antigen to a sample; measuring the amount of the labeling substance released by lysis of the liposome membrane by SLO; and thereby determining ASO value in the sample.

The amounts of the liposomes, labeling substance, SLO, etc. used in the determination method of this invention in terms of their concentration in a total reagent mixture, i.e., a mixture of a sample, a SLO solution and a liposome suspension (or a total reagent mixture before addition of the reaction terminator solution hereinafter described, in the case of determination using the reaction terminator) are as follows: the amount of the liposomes is usually 5 to 500 nmol/ml, preferably 30 to 100 nmol/ml in the total reagent mixture in terms of the amount of phospholipid or glycolipid. The amount of the labeling substance to be encapsulated in the liposomes is varied depending on the kind of the labeling substance, but for example, when the labeling substance is alkaline phosphatase (hereinafter abbreviated as AP), said amount is usually 0.02 to 0.6 unit/ml, preferably 0.1 to 0.2 unit/ml in the total reagent mixture. Although the amount of SLO is not critical because it is varied depending on the kind of the liposome, an amount of 0.5 to 100 $\mu$g/ml in the total reagent mixture is usually preferably employed. When an enzyme is used as the labeling substance in the determination method of this invention, a substrate is used for measuring the amount of the enzyme released from the liposomes. The amount of the substrate used is varied depending on the kinds of the enzyme and substrate used, but for example, when AP is used, said amount is usually 0.5 to 10 mM, preferably 2 to 5 mM in terms of the concentration of p-nitrophenylphosphate as substrate solution.

In the determination method of this invention, a buffer solution is usually used for conducting the determination stably or as a diluent for the liposome suspension, the SLO solution, etc. As the buffer solution, there can be used all the conventional buffer solutions including phosphate buffer, Good's buffer (HEPES, PIPES, MES, etc.), Tris buffer, etc. Water-soluble proteins such as bovine serum albumin (BSA), gelatin and the like, sugars, chelating agents or reducing agents. may be properly added as additives to these buffer solutions. SLO is used usually in the form of an aqueous solution but is relatively unstable in an aqueous solution. Therefore, for stabilizing SLO, it is preferable to incorporate water-soluble proteins such as BSA, gelatin, etc. previously into the buffer solution used for the SLO solution. Although the adding amount of the protein is not critical, it is usually about 0.05 to about 2.0 w/v% in the buffer solution. In the determination method of this invention, ASO value can be accurately determined even when the above-mentioned proteins are added.

The determination method of this invention is described below, for example, by taking the case where liposomes encapsulating AP as a labeling substance are used and p-nitrophenylphosphate is used as a substrate.

First, a sample and SLO are mixed and then pre-incubated for about 1 to about 10 minutes, followed by adding thereto the liposomes; or a sample, SLO and the liposomes are mixed simultaneously. Then, the resulting mixture is incubated for 5 to 30 minutes, after which p-nitrophenylphasphate as substrate is added and the mixture thus obtained is incubated for another 5 to 30 minutes. After the predetermined time, the reaction is terminated by addition of a reaction terminator (e.g., NaOH, KOH, etc.), and absorbance at 410 nm is measured. Alternatively, all the reagents (i.e., SLO, the liposomes and the substrate) are allowed to act on a sample at the same time, and after incubation for 5 to 30 minutes, the reaction is terminated by addition of a reaction terminator and absorbance at 410 nm is measured. Subsequently, the ASO value in the sample is determined from a calibration curve previously prepared by use of standard samples.

When a enzyme is used as a labeling substance, it is also possible to determine the ASO value by measuring the change of absorbance per predetermined time after preincubating the total reagent mixture and a substrate or preincubating the total reagent mixture and thereafter adding the substrate.

Although either of the methods described above may be employed, the latter method in which a sample, SLO and the liposomes are mixed simultaneously is superior because its procedure is simple and the determination can be carried out in a short time.

It is also possible to determine the ASO value by measuring absorbance after substantial completion of the lysis of the liposomes without using a reaction terminator.

For example, when a luminous substance or a dye is used as the labeling substance, the amount of the labeling substance released from the liposomes is determined by measuring absorbance directly without using a substrate. Also when other labeling substances are used, the amount of these substances released can be determined by a well known method for them.

As is clear from the above detailed description, the kit used for the determination method of this invention comprises a suspension of liposomes encapsulating a labeling substance and a solution of SLO.

The solution of SLO preferably contains water-soluble proteins such as BSA, gelatin, etc., particularly preferably BSA.

For example, when an enzyme is used as the labeling substance, in addition to these reagents a substrate solution for the enzyme is used.

The conventional buffer solutions described above also can optionally be added.

As described above in detail, the determination method of this invention is sufficiently applicable not only to a manual method but also to determination using an automatic analyzer, and permits easy and rapid determination. When the determination is carried out by means of an automatic analyzer, combination of reagents, etc. are not critical and there may be properly selected a combination of reagents which seems to be the most suitable in consideration of the type of the automatic analyzer or other factors.

This invention is further explained in more detail with reference to Referential Examples and Examples, which are by way of illustration.

Referential Example 1

Preparation of liposomes encapsulating AP using di-O-hexadecylphosphatidylcholine as a phospholipid

In a test tube were placed 325 µl of a 20 mM solution of di-O-hexadecylphosphatidylcholine in chloroform, 325 µl of a 20 mM solution of cholesterol in chloroform, and 85 µl of a 6 mM solution of dipalmitoylphosphatidylglycerol in chloroform/methanol (95:5), and after mixing, the solvent was distilled off by means of a rotary evaporator. The residue was dried in a desiccator for 2 hours, after which 0.5 ml each of chloroform and diethyl ether were added, followed by adding thereto 80 µl of an alkaline phosphatase (AP) solution [a solution of 5,000 unit/2.5 ml of Ap mfd. by Sigma Chemical Company in 0.01M HEPES (N-2-hydroxyethylpiperazine-N′-ethanesulfonic acid) buffer, pH 7.4], and the resulting mixture was vigorously stirred with a Vortex mixer. The mixture was then concentrated on a water bath at 43° to 48°C by means of a rotary evaporator, and the organic solvent was removed by distillation, after which 1 ml of 0.01M HEPES buffer was added and the resulting mixture was stirred with a Vortex mixer until uniform dispersion was achieved. The dispersion thus obtained was transferred to a centrifuge tube and centrifugation at 34,000 r.p.m. at 4°C for 40 minutes was repeated 5 times to remove the supernatant. Then, the pellet (the precipitation) was suspended in 2 ml of 0.01 M HEPES buffer and the resulting suspension was stored at 4°C.

Referential Example 2

Preparation of liposomes encapsulating AP using LPS as a part of constituents

LPS-liposomes encapsulating AP were prepared in exactly the same manner as in Referential Example 1, except that a mixture of the lipid solutions used in Referential Example 1 and a suspension of 0.5 mg of lipopolysaccharide (LPS) in 1 ml of chloroform/methanol (1:1) was used in place of said lipid solutions.

Referential Example 3

Preparation of liposomes encapsulating AP using LPS and DPPC

In a round bottom flask were placed 2 ml of 20 mM solution of dipalmitoylphosphatidylcholine (DPPC) in chloroform and 2 ml of a 20 mM solution of cholesterol in chloroform, and after mixing, the solvent was distilled off by means of a rotary evaporator. Further, the residue was dried in vacuum by means of a vacuum pump in a desiccator for 2 hours to form a thin film of the lipids on the interior surface of the flask. The thin film was hydrated by addition of 0.6 ml of a 200 mM aqueous n-octyl glucoside solution containing LPS in an amount of 0.6 mg/ml and 0.01 M HEPES buffer (pH 7.4), and the mixture thus obtained was stirred with a Vortex mixer until uniform dispersion was achieved. Then, 250 µl of an aqueous AP solution (2,000 unit/ml) was added, and after stirring and mixing for a while, the resulting mixture was dialyzed against 0.01 M HEPES buffer at 50°C. After about 2 hours, the liposome suspension thus obtained was transferred to a centrifuge tube, and centrifugation at 35,000 r.p.m. at 4°C for 40 minutes was repeated 5 times to remove the supernatant. Then, the pellet was suspended in 0.01 M HEPES buffer and the resulting suspension was stored at 4°C.

Reperential Example 4

Preparation of liposomes encapsulating AP using LPS and egg yolk lecithin

Liposomes encapsulating AP were prepared in exactly the same manner as in Referential Example 3, except that a 1.4 wt% solution of egg yolk lecithin in chloroform was used in place of the DPPC solution and that the temperature at dialysis was changed from 50°C to room temperature.

Referential Example 5

Preparation of liposomes encapsulating AP using LPS, DPPC and egg yolk lecithin

Liposomes encapsulating AP were prepared in exactly the same manner as in Referential Example 3, except that 1.5 ml of a 1.4 wt% solution of egg yolk lecithin in chloroform and 0.5 ml of a 20 mM solution of DPPC in chloroform were used in place of the DPPC solution and that the amount of the aqueous AP solution was changed from 250 µl to 1.5 ml.

Example 1

To 10 ul of the liposome suspension obtained in Referential Example 1 were added 20 µl of each of ASO solutions different in concentration, 20 µl of a SLO solution (concentration: 1 mg/ml) and 400 µl of a 2 mM p-nitrophenylphosphate disodium solution and the resulting mixture was incubated at 37°C for 30 minutes. Then, 1.5 ml of a 0.1N NaOH solution was added to terminate the reaction and absorbance at 410 nm was measured. The result obtained is shown by -○- in Fig. 1.

The amounts of the liposomes, AP and SLO in the total reagent mixture (which did not contain the NaOH solution) were 60 nmol/ml in terms of the amount of phospholipids, 0.6 unit/ml and 44.4 µg/ml, respectively. As is obvious from Fig. 1, a good linear relationship exist between ASO value and absorbance, indicating that the ASO value can be accurately determined by this invention.

Example 2

Determination was carried out in exactly the same manner as in example 2, except that 10 µl of the LPS-liposome suspension obtained in Referential Example 2 was used, to obtain the result shown by -●- in Fig. 1.

Example 3

To 20 µl of each of ASO solutions different in concentration was added 20 µl of the liposome suspension obtained in Referential Example 3, and the resulting mixture was diluted to a volume of 200 µl with 50 mM Tris [tris(hydroxymethyl)-aminomethane]-HCl buffer (pH 7.8). 20 µl of a SLO solution (predetermined concentration) and 400 µl of 2 mM p-nitro-phenylphosphate solution were added to the resulting dilute. After incubation at 37°C for 20 minutes, the reaction was terminated by addition of 2 ml of a 0.1 N NaOH solution and absorbance at 410 nm was measured. The relationship between ASO value (Todd) and relative absorbance (a value determined by taking the absorbance measured for a sample containing no ASO as 100) is shown in Fig. 2. In Fig. 2, -●-. -○- and -△- show the results obtained when an SLO solution having a concentration of 1 mg/ml, 0.5 mg/ml or 0.25 mg/ml, respectively, was used, that is to say, -●-. -○- and -△- show the result obtained when the amount of SLO in the total reagents mixture was 32 µg/ml, 16 µg/ml or 8 µg/ml, respectively.

Example 4

10 µl of the liposome suspension obtained in Referential Example 4 was diluted 20 times with 50 mM Tris-HCl buffer containing 0.1% BSA and gelatin (pH 7.8). To 200 µl of the resulting dilution was added 400 µl of 2 mM p-nitrophenylphosphate disodium solution containing SLO (the amount of SLO: 2 µg) obtained by dilution with the same buffer as described above, and the resulting mixture was incubated at 37°C for 30 minutes. Then 2 ml of a 0.1 N NaOH solution was added to terminate the reaction and absorbance at 410 nm was measured. The result obtained is shown in Fig. 3-(a).

Example 5

Determination was carried out in exactly the same manner as in Example 4, except for changing the BSA content of the buffer solution used for diluting the liposome suspension and the substrate solution containing SLO. The results obtained are shown in Fig. 3 (b), (c) and (d). Fig. 3, (b), (c) and (d) show the results obtained when the BSA content was 0.25%, 0.5% or 1.0%, respectively.

It can be seen that as is clear from these results, the determination method of this invention is not adversely affected by the content of BSA which was added for stabilizing SLO in the diluting buffer solution.

Example 6

ASO value was determined by means of an automatic analyzer Hitachi Model 7050.

First, 5 µl of a sample was mixed with 350 µl of a first reagent R1 [prepared by diluting 2 µl of the liposome suspension obtained in Referential Example 5 to a volume of 350 µl with 50 mM Tris-HCl buffer (containing 0.17% BSA and gelatin)] and correction blank was conducted. After 5 minutes, 250 µl of a second reagent R2 (the amount of SLO: 0.5 µg, substrate concentration: 2 mM) was added. After another 5 minutes, the change of absorbance per minute at 415 nm (700 nm) was measured. A calibration curve for the ASO value in the present method is shown in Fig. 4. In Fig. 4, the axis of abscissa refers to the ASO value (Todd) and the axis of ordinate to the change of absorbance per minute at 415 nm measured by using as a reference a physiological saline solution in place of a sample. The correlation between the present method and a conventional method (Rantz-Randall method) is shown in Fig. 5.

As is obvious from Fig. 5, the values obtained by the determination method of this invention correlate well to those obtained by the conventional method, and therefore this invention permits accurate determination.

## Claims

**1.** A method for determination of antistreptolysin O (ASO) value, which comprises
adding (a) liposomes encapsulating a labeling substance and (b) streptolysin O (SLO) as both antigen and lytic agent to a sample,
measuring the amount of the labeling substance released by lysis of the liposome membrane by SLO, and
thereby determining ASO value in the sample.

**2.** The method according to Claim 1, wherein the labeling substance is selected from enzymes, coenzymes, luminous compounds, fluorescent compounds, dyes, sugars, ionic compounds and radical compounds.

**3.** The method according to Claim 1, wherein the labeling substance is an enzyme.

**4.** The method according to Claim 2, wherein the determination is carried out in the presence of a water-soluble protein.

**5.** The method according to Claim 4, wherein the water-soluble protein is bovine serum albumin (BSA).

**6.** The method according to Claim 1, wherein the liposome contains a lipopolysaccharide (LPS) or a LPS-like compound selected from compounds obtained by subjects LPS to chemical modification and complexes of LPS with polymyxin or a carbocyanine type dye as a part of its constituents.

**7.** A method for determination of ASO value, which comprises
adding (a) a suspension of liposomes encapsulating a labeling substance and (b) a solution of SLO to a sample,
measuring the amount of the labeling substance released by lysis of the liposome membrane by SLO remaining after the antigen-antibody reaction between ASO in the sample and SLO added, and
determining ASO value in the sample from a standard calibration curve previously prepared by conducting determination for standard samples in the same manner as described above.

**8.** The method according to Claim 7, wherein the amount of the lipsomes used for the determination is 5 to 500 nmol/ml in terms of phospholipid or glycolipid in a total reagents mixture (a mixture of a sample, a solution of SLO and a suspension of liposome), and the amount of SLO used therefor is 0.5 to 100 µg/ml in the total reagent mixture.

**9.** The methods according to Claim 7, wherein the solution of SLO contains a water-soluble protein.

**10.** A kit for determining ASO value in a sample which comprises (a) a suspension of liposomes encapsulating a labeling substance and (b) a solution of SLO.

**11.** The kit according to Claim 10, wherein the labeling substance is an enzyme.

**12.** The kit according to Claim 10, which further comprises a substrate solution for the enzyme.

**13.** The kit according to Claim 10, wherein the solution of SLO contains a water-soluble protein.

**14.** The kit according to Claim 10, wherein the liposome contains a lipopolysaccharide (LPS) or a LPS-like compound selected from compounds obtained by subjecting LPS to chemical modification and complexes of LPS with polymyxin or a carbocyanine type dye as a part of its constituents.

**Revendications**

**1.** Procédé de détermination de la valeur de l'anti-streptolysine O (ASO), dans lequel

on ajoute (a) des liposomes encapsulant une substance marquante et (b) de la streptolysine O (SLO) à la fois comme antigène et comme agent lytique à un échantillon,

on mesure la quantité de substance marquante libérée par la lyse de la membrane liposomique par la SLO, et

on détermine ainsi la valeur de l'ASO dans l'échantillon.

**2.** Procédé selon la revendication 1, dans lequel la substance marquante est choisie parmi les enzymes, coenzymes, composés lumineux, composés fluorescents, colorants, sucres, composés ioniques et composés radicalaires.

**3.** Procédé selon la revendication 1, dans lequel la substance marquante est une enzyme.

**4.** Procédé selon la revendication 2, dans lequel la détermination est conduite en présence d'une protéine soluble dans l'eau.

**5.** Procédé selon la revendication 4, dans lequel la protéine soluble dans l'eau est la sérum-albumine de boeuf (SAB).

**6.** Procédé selon la revendication 1, dans lequel le liposome contient un lipopolysaccharide (LPS) ou un composé de type LPS choisi parmi les composés obtenus en soumettant un LPS à une modification chimique et des complexes de LPS avec la polymyxine ou un colorant de type carbocyanine comme partie de ses constituants.

**7.** Procédé de détermination de la valeur de l'ASO, dans lequel

on ajoute (a) une suspension de liposomes encapsulant une substance marquante et (b) une solution de SLO à un échantillon,

on mesure la quantité de la substance marquante libérée par la lyse de la membrane liposomique par la SLO restant après la réaction antigène-anticorps entre l'ASO dans l'échantillon et la SLO ajoutée, et

on détermine la valeur de l'ASO dans l'échantillon à partir d'une courbe d'étalonnage de référence préparée antérieurement en conduisant la détermination pour les échantillons de référence de la même manière qu'il est dit ci-dessus.

**8.** Procédé selon la revendication 7, dans lequel la quantité de liposomes utilisée pour la détermination est de 5 à 500 nmoles/ml en termes de phospholipide ou de glycolipide dans un mélange total de réactifs (un mélange d'un échantillon, d'une solution de SLO et d'une suspension de liposomes), et la quantité de SLO utilisée à cet effet est de 0,5 à 100 $\mu$g/ml dans le mélange réactionnel total.

**9.** Procédés selon la revendication 7, dans lesquels la solution de SLO contient une protéine soluble dans l'eau.

**10.** Equipement pour déterminer la valeur de l'ASO dans un échantillon qui comprend (a) une suspension de liposomes encapsulant une substance marquante et (b) une solution de SLO.

**11.** Equipement selon la revendication 10, dans lequel la substance marquante est une enzyme.

**12.** Equipement selon la revendication 10, qui comprend une solution substrat pour l'enzyme.

**13.** Equipement selon la revendication 10, dans lequel la solution de SLO contient une protéine soluble dans l'eau.

**14.** Equipement selon la revendication 10, dans lequel le liposome contient un lipopolysaccharide (LPS) ou un composé de type LPS choisi parmi les composés obtenus en soumettant le LPS à une modification chimique et des complexes de LPS avec la polymyxine ou un colorant de type carbocyanine en tant que partie de ses constituants.

**Patentansprüche**

**1.** Verfahren zur Bestimmung des Antistreptolysin O (ASO) Wertes, umfassend die folgenden Schritte:

Zugabe von (a) Liposomen, die einen Markierungsstoff einkapseln, und (b) Streptolysin O (SLO) als Antigen und Lyse-Mittel zu einer Probe,

Messen der Menge des Markierungsstoffes, der durch die Lyse der Liposomenmembran durch SLO freigesetzt wurde, und
dadurch Ermitteln des ASO-Wertes in der Probe.

2. Verfahren nach Anspruch 1, dadurch gekennzeichnet, daß der Markierungsstoff ausgewählt ist aus Enzymen, Coenzymen, Leuchtverbindungen, fluoreszierenden Verbindungen, Farbstoffen, Zuckern, Ionenverbindungen und Radikalverbindungen.

3. Verfahren nach Anspruch 1, dadurch gekennzeichnet, daß der Markierungsstoff ein Enzym ist.

4. Verfahren nach Anspruch 2, dadurch gekennzeichnet, daß die Bestimmung durchgeführt wird in Gegenwart eines wasserlöslichen Proteins.

5. Verfahren nach Anspruch 4, dadurch gekennzeichnet, daß das wasserlösliche Protein Rinderserumalbumin (BSA) ist.

6. Verfahren nach Anspruch 1, dadurch gekennzeichnet, daß das Liposom ein Lipopolysaccharid (LPS) oder eine LPS-artige Verbindung enthält, die ausgewählt ist aus Verbindungen, die dadurch erhalten wurden, daß man LPS einer chemischen Modifikation unterzog, und aus LPS-Komplexen mit einem polymyxinartigen oder carbocyaninartigen Farbstoff als Teil ihrer Bestandteile.

7. Verfahren zur Bestimmung des ASO-Wertes, umfassend die folgenden Schritte:
Zugabe von (a) einer Suspension von Liposomen, die einen Markierungsstoff einkapseln, und (b) einer Lösung von SLO zu einer Probe,
Messen der Menge des Markierungsstoffes, der freigesetzt wurde durch die Lyse der Liposomenmembran durch SLO, das nach der Antigen-Antikörperreaktion zwischen dem ASO in der Probe und dem zugegebenen SLO übrigblieb, und
Ermitteln des ASO-Wertes in der Probe anhand einer standardisierten Eichkurve, die zuvor durch die Ermittlung für die Standardproben in der oben beschriebenen Weise hergestellt wurde.

8. Verfahren nach Anspruch 7, dadurch gekennzeichnet, daß die Menge der für die Bestimmung verwendeten Liposomen 5 bis 500 nmol/ml bezogen auf Phospholipid oder Glycolipid in einer totalen Reagenzienmischung (eine Mischung aus einer Probe, einer Lösung von SLO und einer Liposomensuspension) beträgt, und daß die hierfür verwendete Menge von SLO 0,5 bis 100 µg/ml in der totalen Reagenzienmischung beträgt.

9. Verfahren nach Anspruch 7, dadurch gekennzeichnet, daß die Lösung von SLO ein wasserlösliches Protein enthält.

10. Ausrüstung zum Bestimmen des ASO-Wertes in einer Probe, bestehend (a) aus einer Suspension von Liposomen, die einen Markierungsstoff einkapseln, und (b) aus einer Lösung von SLO.

11. Ausrüstung nach Anspruch 10, dadurch gekennzeichnet, daß der Markierungsstoff ein Enzym ist.

12. Ausrüstung nach Anspruch 10, ferner umfassend eine Substratlösung für das Enzym.

13. Ausrüstung nach Anspruch 10, dadurch gekennzeichnet, daß die Lösung von SLO ein wasserlösliches Protein enthält.

14. Ausrüstung nach Anspruch 10, dadurch gekennzeichnet, daß das Liposom ein Lipopolysaccharid (LPS) oder eine LPS-artige Verbindung enthält, die ausgewählt ist aus Verbindungen, die dadurch erhalten wurden, daß man LPS einer chemischen Modifikation unterzog, und aus LPS-Komplexen mit einem polymyxinartigen oder carbocyaninartigen Farbstoff als Teil ihrer Bestandteile.

FIG. 1

ABSORBANCE
1.0
0.5
0
0
500
1000
ASO VALUE (Todd)

FIG. 2

100
50
0
RELATIVE ABSORBANCE (%)
0
500
1000
1500
ASO VALUE (Todd)

F I G. 3
(a)
ABSORBANCE
0.5
0
0
200
400
600
ASO VALUE (Todd)
(b)
ABSORBANCE
0.5
0
0
200
400
600
ASO VALUE (Todd)

F I G. 3
(c)
ABSORBANCE
0.5
0
0
200
400
600
ASO VALUE (Todd)
(d)
ABSORBANCE
0.5
0
0
200
400
600
ASO VALUE (Todd)

FIG. 4

0.10
0.05
CHANGE OF ABSORBANCE PER MINUTE
0
500
ASO VALUE ( Todd )

FIG. 5

625
500
333
250
166
125
100
50
12
12
50
100
125
166
250
333
500
625
ASO VALUE (Todd) BY THE PRESENT METHOD
ASO VALUE (Todd) BY RANTZ-RANDALL METHOD